# EUROPÄISCHE PATENTANMELDUNG

(11) **EP 2 111 827 A1**
(43) Veröffentlichungstag der Anmeldung: **28.10.2009**
(21) Anmeldenummer: 09156304.9
(22) Anmeldetag: 26.03.2009
(51) Int. Cl.: A61F 2/84

(54) **Stentbefestigungssystem**

(30) Priorität: 26.04.2008 DE 102008021066
(71) Anmelder: Biotronik VI Patent AG, 6341 Baar (CH)
(72) Erfinder: Surber, Bettina, 8600 Dübendorf (CH); Wintsch, Daniel, 8052 Zürich (CH); Conzelmann, Tommy, 8200 Schaffhausen (CH); Götzen, Nils, 18057 Rostock (DE); Gerold, Bodo, 91225 Zellingen (DE); Borck, Alexander, 91086 Aurachtal (DE)
(74) Vertreter: Lindner-Vogt, Karin L.

(57) **Zusammenfassung**

Stentbefestigungssystem 3 zur Befestigung eines Stents umfassend zwei Haltebereiche 10, wobei die zwei Haltebereiche 10 durch einen Verbindungsbereich 12 verbunden sind, und somit einen Fixierschlauch formen, und wobei die Haltebereiche 10 eine Haltestruktur bereitstellen, die zur Aufnahme mindestens einer Stentstrebe 2 des Stents im nichtdilatierten Zustand derart ausgebildet ist, dass die Stentstrebe 2 beim Dilatieren des Stents oder zumindest im dilatierten Zustand des Stents sich nicht mehr in einem der Haltebereiche 10 befindet.

## Beschreibung

Die Erfindung bezieht sich auf ein Befestigungssystem für einen Stent.

In zunehmendem Maß werden in der modernen Implantationsmedizin Implantate zur Wiedereröffnung und Stützung von Hohlorganen wie zum Beispiel Blutgefäßen, Harnleitern, Gallengängen, Uteri und Bronchien im menschlichen Körper eingesetzt. Zur Stenose-Behandlung in Blutgefäßen wird in einem medizinischen Eingriff erst eine Ballondilatation durchgeführt und anschließend ein Stent zur Verhinderung einer erneuten Verengung des geweiteten Gefäßes eingesetzt. In einem anderen Verfahren wird die Ballondilatation gleichzeitig mit dem Platzieren des Stents durchgeführt. Hierfür wird ein Einführsystem auf den Ballonkatheter montiert. Mit Hilfe verschiedener Befestigungsmethoden wird ein Stent auf dem Einführsystem befestigt. Nach der Dilatation wird der Ballon aus dem behandelten Gefäß entfernt, wobei der Stent am Ort der Dilatation verbleibt.

Zur Unterstützung der Therapie werden seit einiger Zeit Stents mit Medikamenten beschichtet. Die Medikamente dienen entweder der Vermeidung/Verminderung von entzündlichen Reaktionen der Gefäßwände nach dem Eingriff (antiinflammatorisch) oder der überschießenden Proliferation glatter Gefäßmuskelzellen, was zu einer Restenose führen kann. Manche Substanzen dienen auch der beschleunigten Besiedlung des Stents mit Endothelzellen. Dieser gewünschte Effekt beschleunigt das Einwachsen des Stents in die Gefäßwand.

Der Stent muss so beschaffen sein, dass er einerseits ein Einführen mittels Katheter möglichst leicht macht und andererseits am Ort der Stenose seine Aufgabe der Gefäßabstützung mit entsprechend großer Radialkraft erfüllt. Dafür wird der Stent in einem komprimierten Zustand bis zum Behandlungsort eingeführt und dort dilatiert. Die Dilatation des Stents erfolgt entweder mit Hilfe eines Ballons, auf den der Stent montiert ist und der dann mittels Druck aufgeweitet wird, wobei sich gleichermaßen der Stent aufweitet. Nach Druckablass aus dem Ballon verbleibt der Stent in der dilatierten Struktur im Gefäß. Die Alternative zur Ballondilatation ist die Verwendung eines Stents aus einer Formgedächtnislegierung wie beispielsweise Nitinol. Der komprimierte Stent ist mit einem Außenschlauch überzogen. Nach Einbringen des Stents an den Ort der Stenose wird der Außenschlauch entfernt und der Stent weitet sich auf.

Eine technische Herausforderung bei den selbstexpandierbaren Systemen besteht darin, dass beim Zurückziehen des Außenschlauches ein Stauchen des Stents aufgrund der Wandreibung verhindert wird. Dieses Stauchen entsteht durch die Reibung zwischen Außenschlauch und Stent. Ein gestauchter Stent tendiert dazu, am Außenschaft zu verhaken und erhöht damit die Abziehkräfte des Außenschlauches.

Eine technische Herausforderung an ein ballonexpandierbares System ist, dass das System Stent-Ballon-Katheter so aufeinander abgestimmt ist, dass der Stent während der Einführung in das Gefäß nicht auf dem Einführsystem verrutscht oder gar komplett abrutscht, aber nach Vorbringung an den Behandlungsort ohne Schwierigkeiten freigesetzt werden kann.

Stentbefestigungssysteme sind aus verschiedenen Schriften bekannt; so beispielsweise aus der US 5,776,141. Die US-Schrift beschreibt Haltestrukturen für Stents und andere röhrenförmige Prothesen, insbesondere auf Ballon-Dilatations-Kathetern. Hier wird der Stent mit Hilfe von verschiebbaren Hüllen oder auch ringförmigen, ebenfalls verschiebbaren Halterelementen auf dem Einführsystem fixiert. Ebenfalls beschrieben sind zerreißbare Bänder, die über den Stent gezogen werden. Hier überdeckt das jeweilige Halterelement den gesamten Stent.

Die US 4,950,227 beschreibt ein System, bei dem über das jeweilige Ende des Stents ein Schlauchabschnitt geschoben ist, der den Stent auf dem Ballon fixiert. Nach Vorbringung des Stents an den Behandlungsort bleiben die Schlauchabschnitte trichterförmig geöffnet am Katheter hängen. Diese Tatsache kann dazu führen, dass sich vor oder während des Rückziehens des Kathetersystems aus den Gefäßen die Schlauchabschnitte entweder in der netzartigen Struktur des Stents, in den kalzifizierten Gefäßen oder auch beim Zurückziehen in den Führungskatheter verhaken und somit das Rückziehen stark gefährden. Dies trifft insbesondere für den distalen Schlauchabschnitt zu, da hier die Trichteröffnung in Richtung der Rückzugbewegung des Kathetersystems weist.

Die Patentanmeldung EP 1 785 107 beschreibt ein Haltesystem, bei dem Bänder, offene Laschen oder auch ringförmig umlaufende Stentstreben den Katheter mit dem Stent verbinden. Die relativ langen Bänder haben den Nachteil, dass diese bei Zurückziehen des Kathetersystems in den gekrümmten Gefäßen auf der Kurveninnenseite gestaucht werden und dadurch abstehen. Das führt dazu, dass sie die Gefäßwände berühren und dadurch Gewebeirritationen hervorrufen können.

Die vorbekannten Lösungen haben darüber hinaus den Nachteil, dass an den beiden Stentenden so genanntes Flaring auftritt. Als Flaring bezeichnet man das Abstehen der Stentenden in gekrümmten Gefäßbahnen. Durch seine prinzipiell elastische Struktur in axialer Richtung kann der Stent nahezu jede Krümmung der Gefäßbahnen mitmachen. Allerdings streben beide Stentenden aus rein mechanischen Gründen eine Streckung an. Das bedeutet, dass die Stentenden tendenziell weniger gekrümmt sind. Das bewirkt eine Berührung der Gefäßwände, was einerseits das Vorschieben des Systems behindern und anderseits entzündliche und daher unerwünschte Gewebereaktionen hervorrufen kann.

Die zu lösende Aufgabe ist es daher, ein Stentbefestigungssystem zur Verfügung zu stellen, das ein leichtes Lösen des Stents ermöglicht, und bei dem keine wie auch immer gearteten Halteelemente nach Freisetzen des Stents im Gefäß verbleiben oder beim Rückzug mit der Gefäßwand oder dem Führungskatheter verhaken.

Diese Aufgabe wird mit dem erfindungsgemäßen Stentbefestigungssystem 3 gelöst. Das Stentbefestigungssystem 3 umfasst gemäß Anspruch 1 einen Fixierschlauch, der sich aus zwei Haltebereichen 10 und einem Verbindungsbereich 12 zusammensetzt. Der Verbindungsbereich 12 verbindet die beiden Haltebereiche 10.

Die Haltebereiche 10 stellen eine Haltestruktur bereit, die zur Aufnahme mindestens einer Stentstrebe 2 des Stents im nicht-dilatierten Zustand derart ausgebildet ist, dass die Stentstrebe 2 beim Dilatieren des Stents oder zumindest im dilatierten Zustand des Stents sich nicht mehr in einem der Haltebereiche 10 befindet. Sobald der Stent dilatiert wird und dadurch der Abstand der einzelnen Stentstreben 2 zueinander vergrößert wird, werden Stentstreben 2 und Haltebereiche 10 so zueinander versetzt, dass der Haltebereich 10 die Stentstrebe 2 nicht mehr hält.

Das erfindungsgemäße Stentbefestigungssystem 3 hat den Vorteil, dass es, montiert auf ein Einführsystem, den Stent sicher und gefäßschonend an den Behandlungsort im menschlichen oder tierischen Körper bringt. Damit ist es möglich, den Stent auf dem Stentbefestigungssystem 3 zu fixieren, ohne dass nach Freisetzen des Stents die Haltestrukturen das Zurückziehen des Einführsystems stören oder gar im Körper verbleiben.

Durch die einstückige Ausführung des Fixierschlauchs ist ein geringer Durchmesser des Stentbefestigungssystems 3 gewährleistet, da keine Verbindungsnähte (Schweißnähte, Lötstellen und ähnliches) nötig sind. Somit wird das Rückziehen des Einführsystems erleichtert durch einen geringen Durchmesser des Stentbefestigungssystems 3. Das Einführsystem kann somit aus dem Körper entfernt werden, ohne dass es übermäßige Gefäßirritationen auslöst.

Im nicht-dilatierten Zustand ist der Verbindungsbereich 12 des Fixierschlauchs von den Stentstreben 2 bedeckt. Der Verbindungsbereich 12 kann entweder faltbar sein oder er kann Schlitze aufweisen, die nach der Dilatation des Stents eine netzartige Struktur bilden.

Der Fixierschlauch ist aus einem elastischen Material, vorzugsweise aus der Gruppe der thermoplastischen Polyurethane (TPU) hergestellt. Möglich wäre aber auch Material aus der Gruppe der Polyetherblockamide oder Gummi, insbesondere Silikonkautschuk in Verbindung mit einem Medikament. Der Fixierschlauch ist geringfügig größer als die Stentlänge.

Das erfindungsgemäße Stentbefestigungssystem 3 ist auf einem Dilatationskatheter oder dem Katheterschaft 1 montierbar. Hierbei ist es befestigbar entweder auf dem Ballon, am Ballonhals oder am Katheterschaft.

Darüber hinaus kann das gesamte oder Teile des Stentbefestigungssystems 3 mit einer pharmazeutisch aktiven Substanz beschichtet werden. Vorteilhaft ist es insbesondere, die Haltestrukturen der beiden Haltebereiche 10 zu beschichten, da diese am ehesten Kontakt mit den Gefäßwänden 4 haben und dort ihre pharmazeutische Substanz freisetzen können.

Die pharmazeutisch aktive Substanz wird mittels Tauchen, Dippen, Sprühen, Rakeln, Eindiffundieren, Bestreichen oder Pressen auf das Stentbefestigungssystem 3 aufgebracht. Sie kann entweder allein oder aber zusammen mit einem Träger oder Carrier aufgetragen werden.

Durch eine angepasste Geometrie mit relativ kurzen Haltebereichen 10 erreicht man zusätzlich eine höhere seitliche Stabilität des Haltebereichs 10. Diese Stabilität führt zu einem kontrollierteren bzw. reproduzierbaren Freisetzen des Stents am Behandlungsort.

### Es zeigen schematisch:

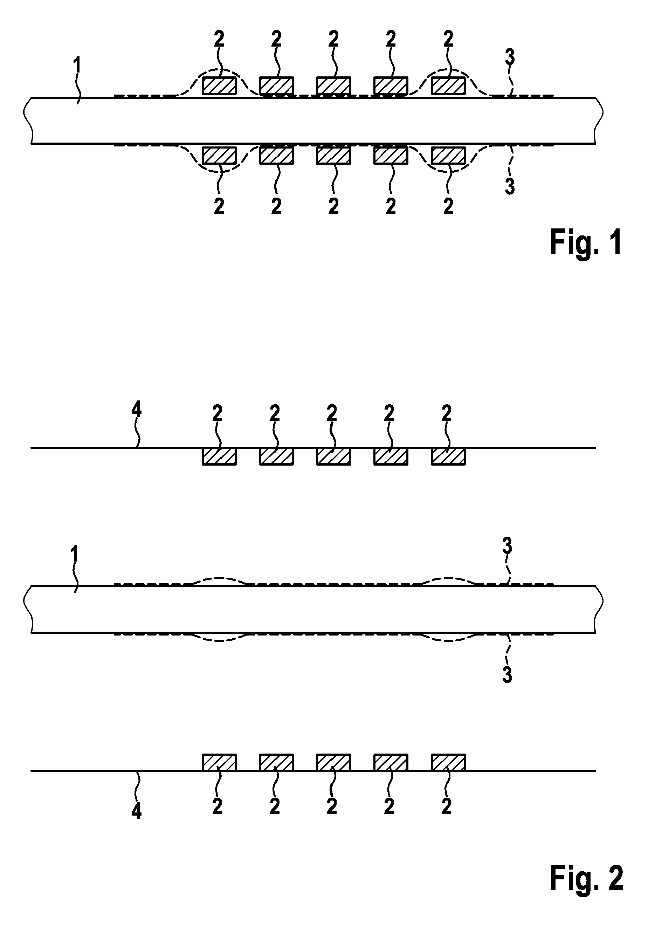

- Fig. 1:: das Stentbefestigungssystem 3, welches den Stent 2 auf dem Innenschaft 1 eines Katheters hält; und zwar im nicht-dilatierten Zustand.
- Fig. 2:: das Stentbefestigungssystem 3, welches den Stent 2 nicht mehr hält. Der dilatierte Stent 2 liegt an der Gefäßwand 4 an.

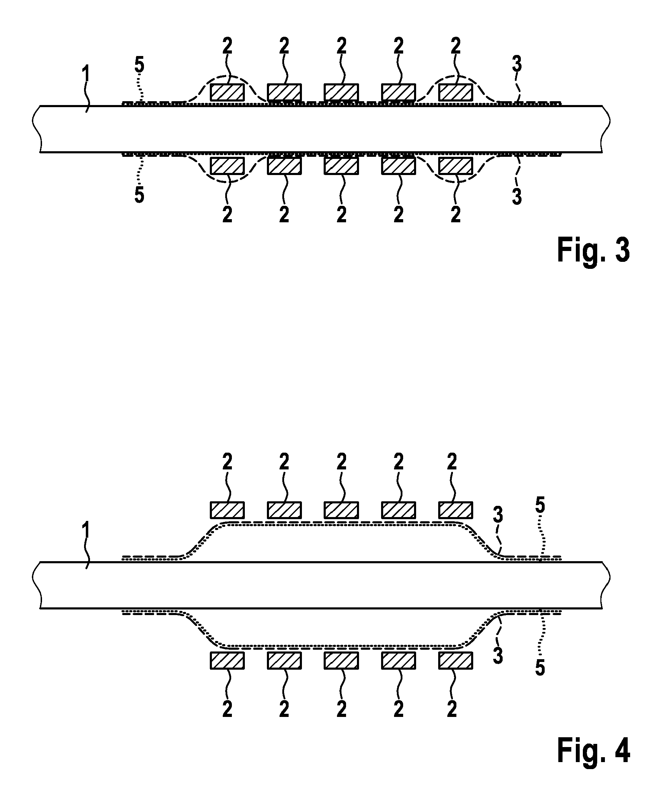

- Fig. 3:: das Stentbefestigungssystem 3 montiert auf einem Dilatationsballon 5. Das Stentbefestigungssystem 3 hält den Stent 2 auf dem Dilatationsballon 5. Das Stentbefestigungssystem 3 und der Dilatationsballon 5 sind auf dem Innenschaft 1 eines Katheters montiert.
- Fig. 4:: das Stentbefestigungssystem 3 mit Dilatationsballon 5 im dilatierten Zustand.

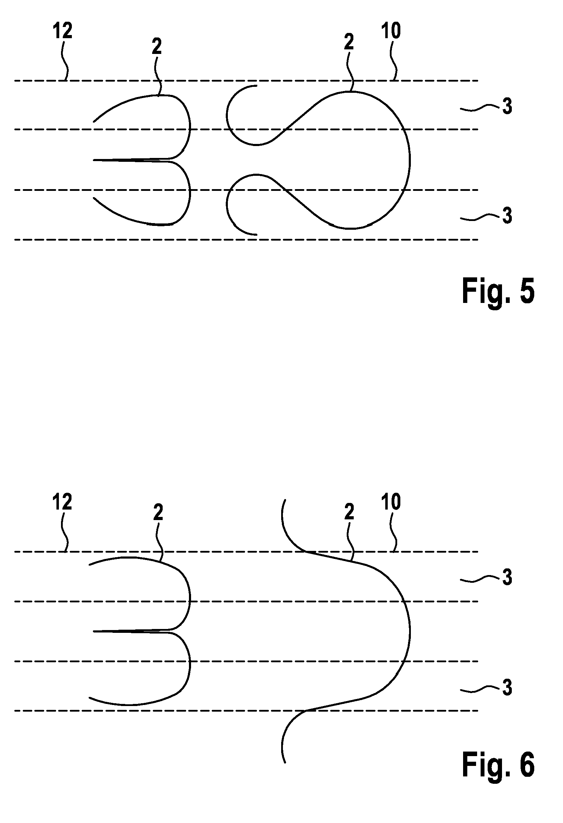

- Fig. 5:: das Stentbefestigungssystem 3 in der Draufsicht, mit einem nicht-dilatierten Stent 2. Die Haltebereiche 10 und der Verbindungsbereich 12 sind dargestellt.
- Fig. 6:: das Stentbefestigungssystem 3 in der Draufsicht, mit einem dilatierten Stent 2. Die Haltebereiche 10 und der Verbindungsbereich 12 sind dargestellt.

Die Erfindung wird anhand eines Ausführungsbeispiels näher erläutert. Diese bevorzugte Ausführung weist einen Fixierschlauch auf, der auf einem Katheter montiert ist. Dieser Katheter weist an seinem distalen Ende einen Dilatationsballon 5 für die Ballondilatation eines Stents auf.

Der Fixierschlauch kann beispielsweise am Katheterschaft, am Ballonhals oder am Ballonzylinder direkt befestigt werden. In Abhängigkeit der Materialkombination zwischen Fixierschlauch und Schaft ist ein geeignetes Verbindungsverfahren (Kleben, Schweißen, Nieten) anzuwenden. Der Stent ist derart auf dem Fixierschlauch positioniert, dass er in den beiden Haltebereichen 10 von der Haltestruktur fixiert wird und der Verbindungsbereich 12 unter dem Stent liegt.

Der Fixierschlauch ist aus einem elastischen Material, vorzugsweise aus der Gruppe der thermoplastischen Polyurethane (TPU) hergestellt. Möglich wäre aber auch Material aus der Gruppe der Polyetherblockamide oder Gummi, insbesondere Silikonkautschuk.

Die beiden Haltebereiche 10 an den jeweiligen Enden des Fixierschlauchs sind durch Schlitzen des Schlauches in definierter Form festgelegt. Die Schlitze des Schlauches bringen bandartige Schlauchteile hervor, die der Fixierung eines oder mehrerer Stentstreben 2 mittels Klemmkräften dient, indem diese Bänder über die Stentstreben 2 verlaufen.

Der Verbindungsbereich 12 des Schlauches weist keine derartigen Schlitze auf und läuft unterhalb der Stentstreben 2 entlang, was die Fixierung bewirkt. Auf diese Weise wird sichergestellt, dass der Stent beim Einführen in den menschlichen oder tierischen Körper und auf seinem Weg durch die Gefäßbahnen nicht auf dem Katheter verrutscht oder gar verloren geht.

Hat der Katheter mit dem gesamten Stent-System den Ort der Behandlung erreicht, wird der Dilatationsballon 5 in vorbekannter Weise durch eine mit Druck eingeführte Flüssigkeit aufgeweitet. Da der Fixierschlauch mitsamt dem Stent fest mit dem Dilatationsballon 5 verbunden ist, erfährt er die gleiche Radialkraft und wird zusammen mit dem Dilatationsballon 5 aufgeweitet.

Im Laufe oder zumindest am Ende der Dilatation, d. h. wenn der Dilatationsballon 5 seinen maximalen Durchmesser erreicht hat, ist der Stent derart aufgeweitet, dass die Haltestrukturen der Haltebereiche 10 den Stent nicht mehr halten, sondern freigeben. Der zunehmend größer werdende Abstand der Stentstreben 2 zueinander bewirkt ein Durchrutschen der Bänder im Haltebereich 10 des Fixierschlauches. Die Breite der Bänder ist darauf ausgelegt, dass der Stent möglichst frühzeitig, also zu Beginn der Dilatation freigegeben wird.

Als Alternative kann der Verbindungsbereich 12 des Schlauches ebenfalls Schlitze aufweisen. Einige Schlitze an den jeweiligen Enden des Fixierschlauches dienen der Stentbefestigung. Die Schlitze im Verbindungsbereich 12 des Schlauches liegen unter dem Stent und haben damit keine Haltewirkung. Sie können durchaus andere Maße aufweisen als die Schlitze im Haltebereich 10. So können sie beispielsweise länger sein.

Beim Dilatieren des Ballons und damit des Schlauches ergibt sich eine netzartige Struktur auch des Verbindungsbereiches 12. Das hat den Vorteil der erhöhten Elastizität des Fixierschlauches. Bei Druckablass aus dem Dilatationsballon 5 zieht sich der Schlauch in eine Form mit geringerem radialem Durchmesser zurück und erleichtert so das Zurückziehen des Kathetersystems.

Die Schlitze können durch verschiedene Schneidverfahren in den Fixierschlauch gebracht werden, so etwa durch Stanzen, Laserschneiden oder Wasserstrahlschneiden. Die Herstellungsart wählt der Fachmann entsprechend des verwendeten Materials und der erwünschten Größe der Schlitze. Alternativ kann die Schlitzstruktur bei der Herstellung des Fixierschlauchs umgesetzt werden, beispielsweise durch entsprechend ausgelegtes Spritzgießen.

Der Fixierschlauch wird mittels Kleben, Schweißen oder Nieten am distalen und proximalen Hals des Dilatationsballons 5 auf dem Katheter befestigt. In Abhängigkeit der Materialkombination zwischen Fixierschlauch und Schaft ist ein geeignetes Verbindungsverfahren (Kleben, Schweißen, Nieten) anzuwenden. Bei Verwendung eines selbstexpandierenden Stents wird der Fixierschlauch auf den Katheterinnenschaft 1 ebenfalls mittels Kleben, Schweißen oder Nieten unter Beachtung der Materialverträglichkeit befestigt.

Zur Implantation eines selbstexpandierbaren Stents wird der Fixierschlauch direkt auf den Innenschaft 1 des Katheters montiert und mit diesem durch Kleben, Schweißen oder Nieten fest verbunden. Der ebenfalls über die gesamte Länge oder nur in den Haltebereichen 10 geschlitzte Schlauch besteht aus einem Material mit hoher Festigkeit, aber mit geringem elastischen Dehnungsvermögen, wie beispielsweise Polyamid oder ein ähnlicher Thermoplast (HDPE).

Die in Richtung der Gefäßwände 4 zeigende Kraft kommt allein aus den elastischen Rückstellkräften des Stentmaterials. Nach Zurückziehen des Außenschlauches über den Stent weitet dieser sich aus und legt sich eng an die Gefäßwände 4. Dabei verlässt der Stent bereits zu Beginn der Dilatation die Haltebänder des Haltebereichs 10. Hier hat der Fixierschlauch die gewünschte Eigenschaft, den Stent vor Stauchen während des Abziehens des Außenschlauches zu schützen.

Eine weitere Ausführungsform betrifft die Ausgestaltung des Verbindungsbereiches 12 des Fixierschlauchs. Um im Zuge einer Ballondilatation die nötigen Durchmesser zu erreichen wird der Verbindungsbereich 12 ähnlich wie der Dilatationsballon 5 in Falten unter den Stent gelegt. Beim Dilatieren weitet er sich und die Falten geben den nötigen Materialbedarf frei. Am Ende der Dilatation kommt der Fixierschlauch direkt über dem Dilatationsballon 5 zu liegen.

Das Stentbefestigungssystems 3 kann mit einer pharmazeutisch aktiven Substanz beschichtet werden. Die pharmazeutisch aktive Substanz ist ein Wirkstoff, insbesondere zur Therapie oder Prophylaxe der In-Stent-Restenose, ausgewählt aus der Gruppe bestehend aus:
- Lipidregulatoren (Fibrate),
- Immunsuppressiva,
- Vasodilatatoren (Sartane),
- Kalziumkanalblocker,
- Calcineurininhibitoren (Tacrolimus),
- Antiflogistika (Cortison, Dichlofenac),
- Antiinflammatorika (Imidazole),
- Antiallergika,
- Oligonukleotide (dODN),
- Östrogene (Genistein),
- Endothelbildner (Fibrin),
- Steroide,
- Proteine / Peptide,
- Proliferationshemmer (Paclitaxel, Sirolimus),
- Analgetika,
- Antirheumatika
- Angiogeneseinhibitoren und
- Zytostatika.

Die Aufbringung der pharmazeutisch aktiven Substanz auf das System geschieht beispielsweise mittels Tauchen (längeres Eintauchen und getaucht Halten, was wahlweise wiederholt werden kann), Dippen (kurzes Eintauchen und sofortiges wieder Herausziehen, das wahlweise auch wiederholt werden kann), Sprühen, Rakeln, Eindiffundieren, Bestreichen oder Pressen.

Die mindestens eine pharmazeutisch aktive Substanz wird vorzugsweise zusammen mit einem Träger oder Carrier (z. B. einem Polymer und/oder einem Lösungsmittel) aufgetragen. Im Fall der Verwendung eines Lösungsmittel verdampft dieses beim und/oder nach dem Auftragen. Beim Austreiben des Lösungsmittels härtet gleichzeitig der Polymer-Carrier aus. Als Lösungsmittel kommen, abhängig von der pharmazeutisch aktiven Substanz, Alkohole und sonstige Stoffe in Betracht, so zum Beispiel Dimethylsulfoxid (DMSO), Aceton, Ether (Di-ethylether), Methanol, Isopropanol und Ester.

Hierbei ist es von Vorteil, wenn die pharmazeutisch aktive Substanz mit oder ohne Carrier nur auf den Haltebereichen 10 des Stentbefestigungssystems 3 aufgetragen ist, da diese am ehesten Kontakt mit der Gefäßwand 4 haben.

Da das Stentbefestigungssystem 3 nur eine kurze Zeitspanne im Körper verbleibt und folglich die Substanzen auf den Haltestrukturen der Haltebereiche 10 nur eine kurze Einwirkzeit haben, ist es von Vorteil, wenn das System mit einer leicht abwaschbaren Beschichtung, beispielsweise aus der Gruppe der Zucker und Fette versehen wird.

Eine besonders bevorzugte Ausführungsform ist die Herstellung des Stentbefestigungssystems 3 aus Silikonkautschuk (Liquid Silicon Rubber, LSR). Die pharmazeutisch aktive Substanz wird ausgewählt aus der Gruppe bestehend aus Paclitaxel, Pimecrolimus und Dexamethason.

Das Zweikomponenten-Silikonmaterial "Siliconrubber" von Dow Corning (oder Bayer), zum Beispiel Silastic® 7-6840, wird 1:1 gemischt und bei 100°C für 20 Minuten zur Reaktion gebracht. Die Reaktion kann im Temperaturbereich von 140 - 170°C gefahren werden. Bei diesen Temperaturen beträgt die Reaktionszeit bis zum Aushärten eine Minute.

Temperaturen von 100°C benötigen längere Aushärtzeiten. Geringere Temperaturen sind bei Verwendung von pharmazeutisch aktiven Substanzen vorzuziehen.

Die Wirkstoffbeladung geschieht, indem der Wirkstoff in einer der beiden Komponenten gelöst bzw. feinst suspendiert wird. Beladungen von bis zu 33% Dexamethason Acetat sind im Liquid Silicon Rubber realisierbar.

Andere Materialien für die Herstellung eines wirkstoffbeladenen Stentbefestigungssystems 3 sind beispielsweise:
- Poly[ethylen-co-(vinyl acetat)]
- Acryl-Polymere wie Poly(methacrylat) Eudragit®, PMMA
- PEO/PEG
- HPMA (N-(2-hydroxypropyl)methacryamid)
- Polyanhydride (Septacin®)
- Polyester wie PLLA, P₄HB, P₃HB, PLGA
- Poly(orthoester)
- Poly(phosphoester).

## Patentansprüche

1. Stentbefestigungssystem 3 zur Befestigung eines Stents umfassend zwei Haltebereiche 10
**dadurch gekennzeichnet,**
**dass** die zwei Haltebereiche 10 durch einen Verbindungsbereich 12 verbunden sind, und mit diesem zusammen einen Fixierschlauch formen,
wobei die Haltebereiche 10 eine Haltestruktur bereitstellen, die zur Aufnahme mindestens einer Stentstrebe 2 des Stents im nicht-dilatierten Zustand derart ausgebildet ist, dass die Stentstrebe 2 beim Dilatieren des Stents oder zumindest im dilatierten Zustand des Stents sich nicht mehr in einem der Haltebereiche 10 befindet.

2. Stentbefestigungssystem 3 nach Anspruch 1, **dadurch gekennzeichnet, dass** der Fixierschlauch aus Materialien der Gruppe der thermoplastischen Polyurethane (TPU) hergestellt ist.

3. Stentbefestigungssystem 3 nach einem der Ansprüche 1 oder 2, **dadurch gekennzeichnet, dass** der Verbindungsbereich 12 Schlitze aufweist, die nach der Dilatation des Stents eine netzartige Struktur bilden.

4. Stentbefestigungssystem 3 nach einem der Ansprüche 1 bis 3, **dadurch gekennzeichnet, dass** der Verbindungsbereich 12 faltbar ist.

5. Stentbefestigungssystem 3 nach einem der vorhergehenden Ansprüche 1 bis 4, **dadurch gekennzeichnet, dass** es auf einen Dilatationskatheter oder auf einen Katheter-Innenschaft 1 montierbar ist.

6. Stentbefestigungssystem 3 nach einem der vorhergehenden Ansprüche 1 bis 5, **dadurch gekennzeichnet, dass** die Gesamtlänge des Fixierschlauches geringfügig größer ist als die Stentlänge.

7. Stentbefestigungssystem 3 nach einem der vorhergehenden Ansprüche 1 bis 6, **dadurch gekennzeichnet, dass** es entweder auf dem Ballon, am Ballonhals oder am Katheterschaft befestigbar ist.

8. Stentbefestigungssystem 3 nach einem der vorhergehenden Ansprüche 1 bis 7, **dadurch gekennzeichnet, dass** mindestens eine pharmazeutisch aktive Substanz auf der Haltestruktur mindestens eines Haltebereichs 10 oder auf dem gesamten Stentbefestigungssystem 3 angeordnet ist.

9. Stentbefestigungssystem 3 nach Anspruch 8, **dadurch gekennzeichnet, dass** die mindestens eine pharmazeutisch aktive Substanz mittels Tauchen, Dippen, Sprühen, Rakeln, Eindiffundieren, Bestreichen oder Pressen aufgebracht ist.

10. Stentbefestigungssystem 3 nach Anspruch 8, **dadurch gekennzeichnet, dass** die mindestens eine pharmazeutisch aktive Substanz zusammen mit einem Träger oder Carrier aufgetragen wird.
